# EUROPÄISCHE PATENTSCHRIFT

(11) **EP 1 731 111 B1**
(45) Veröffentlichungstag und Bekanntmachung des Hinweises auf die Patenterteilung: **19.03.2008**
(21) Anmeldenummer: 05011788.6
(22) Anmeldetag: 01.06.2005
(51) Int. Cl.: A61B 19/00

(54) **Inverse Planung der Katheterisierung**
Inverse planning of catheterisation
Planification inverse de cathéterisation

(43) Veröffentlichungstag der Anmeldung: 13.12.2006
(73) Patentinhaber: BrainLAB AG, 85551 Kirchheim/Heimstetten (DE)
(72) Erfinder: Hartlep, Andreas, Dr., 83629 Naring (DE); Pedain, Christoph, Dr., 81667 München (DE)
(74) Vertreter: Schwabe - Sandmair - Marx

(56) Entgegenhaltungen:
- EP-A- 1 103 229
- EP-A- 1 374 790
- WO-A-97/31581
- US-A- 5 638 819

## Beschreibung

Die vorliegende Erfindung betrifft ein Verfahren und eine Vorrichtung zur Bestimmung und zur Darstellung möglicher Eintritts- und/oder Zielgebiete zur Platzierung eines oder mehrerer Katheter in einem Gebiet, insbesondere in Geweberegionen, wie einem Gehirn, wobei die Bestimmung auf der Basis von Struktur und/oder Anatomiedaten des Gebiets erfolgt.

Bei bekannten Verfahren zur Platzierung eines oder mehrerer Katheter werden die Katheter anhand einfacher Katheterplatzierungsrichtlinien platziert. Bei einer Befolgung dieser Richtlinien wird die Katheterspitze häufig ausreichend tief in einer Gehirnregion platziert und mit einem ausreichenden Abstand von Oberflächen platziert, um den Ausfluss des Infusionsmittels in Aushöhlungen, Sulci oder zurück in die Hirnrinde unwahrscheinlicher zu machen. Die vorgegebenen Richtlinien sind jedoch individuell auf verschiedene Patienten und Geweberegionen abgestimmt, so dass keine gleichmäßige Ausbreitung des Infusionsmittels in beliebige Regionen gewährleistet werden kann und auch ein Ausfluss nicht weit genug verhindert werden kann. Weiterhin ist die Umsetzung der Richtlinien in einer individuellen Patientenanatomie bei der Planung einer oder mehrerer Katheter kompliziert und zeitaufwändig, da für jeden Kathetereintrittspunkt und für jeden entsprechenden Katheterendpunkt die entsprechenden Richtlinien per Hand beispielsweise mit einem geeigneten Computerprogramm ausgemessen und überprüft werden.

Die EP 1 374 790 A1 beschreibt ein Verfahren und eine Vorrichtung zur Vorbereitung einer Drainage, wobei hierzu mindestens ein Katheter an einem Körper positioniert wird, wobei körperspezifisch eine anatomische und/oder Gewebestruktur ermittelt wird, die Lage einer Flüssigkeit im Körper ermittelt wird, und der mindestens eine Katheter unter Verwendung dieser Information so positioniert wird, dass mindestens ein Teil der Flüssigkeit aus dem Körper abgelassen werden kann.

Es ist eine Aufgabe der vorliegenden Erfindung, ein Verfahren und eine Vorrichtung zur Bestimmung und Darstellung möglicher Eintritts- und/oder Zielgebiete für Katheterplatzierungen in einem Gebiet auf der Basis von Struktur- und/oder Anatomiedaten des Gebiets vorzuschlagen, mit welchen eine gezielte und sichere Verabreichung und eine gleichmäßige Ausbreitung des Infusionsmittels wahrscheinlicher wird.

Diese Aufgabe wird durch den Gegenstand der unabhängigen Ansprüche gelöst. Vorteilhafte Ausführungsformen ergeben sich aus den Unteransprüchen. Erfindungsgemäß kann aus einem vorgegebenen Eintrittsgebiet des Katheters ein oder mehrere Zielgebiete einer oder mehrerer Katheter ermittelt werden und umgekehrt kann aus einem vorgegebenen Zielgebiet eines Katheters ein bevorzugtes Eintrittsgebiet eines oder mehrerer Katheter bestimmt werden und es können die ermittelten Gebiete auch zusammen mit den Struktur- und/oder Anatomiedaten dargestellt werden.

Dabei wird beispielsweise von einem Computerprogramm für jeden eingegebenen Eintritts- oder Zielpunkt unter Beachtung vorgegebener oder errechneter Richtlinien beispielsweise zur Katheterplatzierung eine Menge geeigneter Ziel- oder Eintrittspunkte ermittelt, wobei entsprechende Richtlinien beispielsweise zur Katheterplatzierung halbautomatisch umgesetzt werden.

Das erfindungsgemäße Verfahren zur Bestimmung und zur Darstellung möglicher Eintritts- und/oder Zielgebiete für die Platzierung eines oder mehrerer Katheter in einem Gebiet, wie zum Beispiel einem bestimmten Gewebe, wie einem Gehirn oder einer Gehirnregion oder einem Körperteil, wird auf der Basis von funktionellen Daten und/oder Struktur- und/oder Anatomiedaten des Gebiets, wie dem Hirn oder einer Hirnregion, durchgeführt. Die funktionellen Daten und/oder Struktur- und/oder Anatomiedaten des Gebiets können mit beliebigen Bildgebungsverfahren, insbesondere medizintechnischen Bildgebungsverfahren, wie Computertomographie, Kernspintomographie, Ultraschall, Positronen-Emissions-Tomographie oder Single Photon Emission Computed Tomography (SPECT) erfasst werden und können insbesondere vor oder während der Durchführung des erfindungsgemäßen Verfahrens ermittelt werden. Erfindungsgemäß wird mindestens ein Eintrittsgebiet oder eine Eintrittsregion des Katheters in dem Gebiet, wie zum Beispiel dem Hirn oder einer Hirnregion, beispielsweise mittels einer Dateneingabevorrichtung, wie einer Tastatur, einer Maus oder einem Touchscreen, eingegeben oder es wird mindestens ein Zielgebiet oder eine Zielregion des Katheters in dem Gebiet, wie dem Gehirn oder der Gehirnregion, von einem Benutzer angegeben oder vorgegeben.

Unter Berücksichtigung der zum Beispiel zuvor ermittelten funktionellen Daten und/oder Struktur- und/oder Anatomiedaten des Gebiets und unter Berücksichtigung z.B. vorgegebener oder ausgewählter Katheterplatzierungsrichtlinien, wie zum Beispiel bestimmter Abstandscharakteristika des Katheters oder mehrerer Katheter zueinander oder zu charakteristischen Gebieten, wie einer Oberfläche oder bestimmten Gewebeabschnitten des Gebiets oder der Abstand zu Gefäßen und/oder Risikostrukturen, kann aus dem mindestens einen angegebenen oder vorgegebenen, zum Beispiel von einem Benutzer gewählten oder zufällig oder automatisch bestimmten, Eintrittsgebiet mindestens ein Zielgebiet des Katheters oder der Katheterspitze oder eines Katheterabschnittes ermittelt werden. Dabei ist das Eintrittsgebiet des Katheters der Bereich, in welchem der Katheter in das Gebiet eingestochen oder eingeführt wird und das Zielgebiet ist der Bereich, zu welchem oder in dessen Nähe der Katheter oder die Katheterspitze oder ein definierter Katheterabschnitt schließlich gelangen soll, um zum Beispiel die Ausschüttung eines Infusionsmittels in das Zielgebiet, wie Krebszellen oder Tumore, zu gewährleisten.

Umgekehrt kann auch anstelle des Eintrittsgebiets des Katheters oder der Katheterspitze das Zielgebiet zum Beispiel automatisch ermittelt und vorgegeben oder von einem Benutzer angegeben werden, wobei aus dem mindestens einen angegebenen Zielgebiet unter Berücksichtigung der festgelegten oder vorgegebenen Katheterplatzierungsrichtlinien und der ermittelten funktionellen Daten und/oder Struktur- und/oder Anatomiedaten des Gebiets mindestens ein geeignetes und/oder mögliches oder bestmögliches Eintrittsgebiet ermittelt wird. Werden zum Beispiel auf einer Datenausgabevorrichtung, wie einem Bildschirm oder einem Touchscreen, die ermittelten oder bekannten funktionellen Daten und/oder Struktur- und/oder Anatomiedaten dargestellt und ein bevorzugtes Eintrittsgebiet des Katheters angegeben oder ausgewählt und auf der Datenausgabevorrichtung dargestellt, können nach der Ermittlung des zugehörigen oder bestmöglichen Zielgebiets oder nach der Ermittlung aller möglichen Zielgebiete die ermittelten Zielgebiete oder das ermittelte Zielgebiet auf der Datenausgabevorrichtung, wie einem Touchscreen, dargestellt werden, so dass zum Beispiel bei der Ausgabe oder Darstellung von mehreren möglichen Zielgebieten ein oder mehrere bevorzugte Zielgebiete, z.B. von einem Benutzer, ausgewählt werden könnten. Umgekehrt können auch die funktionellen Daten und/oder Struktur- und/oder Anatomiedaten des Gebiets und ein ausgewähltes oder angegebenes Zielgebiet dargestellt werden, wobei nach der Ermittlung oder Berechnung des mindestens einen zugehörigen oder korrespondierenden oder geeigneten oder möglichen oder bestmöglichen Eintrittsgebiets das ermittelte Eintrittsgebiet oder die Eintrittsgebiete oder die Eintrittsregion insbesondere grafisch dargestellt werden.

Auch können zusätzlich zu den dargestellten Informationen, wie dem mindestens einen angegebenen oder ermittelten Eintrittsgebiet oder dem mindestens einen angegebenen oder ermittelten Zielgebiet oder den Struktur- und/oder Anatomiedaten des Gebiets, die bei der Bestimmung der Zielgebiete oder Eintrittsgebiete des Katheters berücksichtigten Katheterplatzierungsrichtlinien, wie beispielsweise vorgegebene oder festgelegte Abstandscharakteristika der Katheter zueinander oder Abstände der Katheter oder der Katheterspitze oder Katheterabgabeabschnitte zu charakteristischen Gebieten, mit dargestellt werden.

Vorzugsweise können auch als Eintrittsgebiet oder Eintrittsgebiete ein Eintrittspunkt oder Eintrittspunkte gewählt oder ermittelt werden, wobei die Eintrittspunkte zum Beispiel innerhalb eines Eintrittsgebiets liegen können. Auch können anstelle oder zusätzlich zu den Zielgebieten ein Zielpunkt oder Zielpunkte angegeben oder ermittelt werden. Dabei können insbesondere ein einzelner oder mehrere einzelne Eintrittspunkte vorgegeben werden, woraus unter Berücksichtigung der Struktur und/oder Anatomie des Gebiets sowie unter Berücksichtigung der Katheterplatzierungsrichtlinien ein oder mehrere korrespondierende oder geeignete oder bestmögliche Zielpunkte ermittelt werden können. Vorzugsweise können auch ein oder mehrere Zielgebiete zum Beispiel von einem Benutzer ausgewählt oder vorgegeben werden, aus welchem bzw. aus welchen, vorzugsweise unter Berücksichtigung der speziellen Anatomie und/oder Struktur und/oder Funktion des Gebiets und unter Berücksichtigung der festgelegten oder vorgegebenen Katheterplatzierungsrichtlinien, ein oder mehrere zugehörige oder mögliche oder geeignete Eintrittspunkte ermittelt werden.

Bevorzugt können auch der mindestens eine Eintrittspunkt oder das mindestens eine Eintrittsgebiet iterativ aus dem mindestens einen angegebenen Zielpunkt bzw. Zielgebiet berechnet werden oder umgekehrt können iterativ der mindestens eine Zielpunkt oder das mindestens eine Zielgebiet aus dem mindestens einen Eintrittspunkt bzw. Eintrittsgebiet bestimmt werden.

So kann zum Beispiel zunächst mindestens ein Eintrittsgebiet des Katheters angegeben werden, zu welchem ein oder mehrere zugehörige oder mögliche Zielgebiete oder Zielpunkte mit Hilfe der festgelegten oder ausgewählten Katheterplatzierungsrichtlinien und der ermittelten Struktur und/oder Funktion und/oder Anatomie des Gebiets ermittelt werden können. Insbesondere können der oder die bestmöglichen oder am besten geeigneten Zielgebiete oder Zielpunkte dargestellt werden oder es können alle möglichen Zielgebiete oder Zielpunkte dargestellt werden, so dass zum Beispiel ein oder mehrere bestmögliche oder am besten geeignete Zielgebiete oder Zielpunkte besonders hervorgehoben werden können oder aus der Menge der vorgeschlagenen Zielgebiete oder Zielpunkte ein Benutzer die gewünschten oder bevorzugten ein oder mehreren Zielpunkte oder Zielgebiete auswählen kann.

Nach der Ermittlung des oder der mindestens einen möglichen oder bestmöglichen Zielgebiete oder Zielpunkte aus dem mindestens einem Eintrittsgebiet oder Eintrittspunkt können zum Beispiel alle zu dem mindestens einen ermittelten Zielgebiet oder Zielpunkt korrespondierenden oder unter Berücksichtigung der Katheterplatzierungsrichtlinien und der ermittelten funktionellen Daten und/oder Struktur- und/oder Anatomiedaten des Gebiets möglichen oder bevorzugten Eintrittsgebiete oder Eintrittspunkte berechnet werden, wobei die ermittelten Eintrittsgebiete oder Eintrittspunkte vorzugsweise innerhalb des zunächst angegebenen oder innerhalb der zunächst angegebenen oder vorgegebenen Eintrittsgebiete liegen. Auch können alle möglichen oder zu den ermittelten Zielgebieten oder Zielpunkten korrespondierenden Eintrittspunkte ermittelt werden, wobei insbesondere nur die Eintrittspunkte angezeigt oder dargestellt werden, welche innerhalb des zunächst angegebenen Eintrittsgebiets oder innerhalb der vorgegebenen Eintrittsgebiete liegen oder welche in einem bestimmten Bereich um die zunächst angegebenen Eintrittsgebiete liegen. Auch können zum Beispiel alle möglichen oder zu dem Zielpunkt oder den Zielpunkten korrespondierenden Eintrittspunkte unter Berücksichtigung geeigneter Richtlinien angezeigt werden, wobei vorzugsweise nur der oder die am besten geeigneten Eintrittspunkte besonders hervorgehoben oder dargestellt werden. Auch können alle möglichen Eintrittspunkte ermittelt werden aber nur ein oder mehrere unter Berücksichtigung der Katheterplatzierungsrichtlinien und der ermittelten Struktur und/oder Funktion und/oder Anatomie des Gebiets am besten geeigneten Eintrittspunkte dargestellt werden.

Auch kann umgekehrt zum Beispiel zunächst mindestens ein Zielgebiet oder Zielpunkt des Katheters von einem Benutzer ausgewählt oder unter Berücksichtigung charakteristischer Größen vorgegeben werden, wobei zu jedem der vorgegebenen Zielgebiete oder Zielpunkte oder auch nur zu einem Teil der vorgegebenen Zielgebiete oder Zielpunkte ein oder mehrere Eintrittsgebiete oder Eintrittspunkte ermittelt werden können, wobei bei der Berechnung oder Ermittlung insbesondere die Katheterplatzierungsrichtlinien und die ermittelte oder bekannte Struktur und/oder Funktion und/oder Anatomie des Gebiets berücksichtigt wird. Zu dem mindestens einen ermittelten möglichen Eintrittsgebiet oder Eintrittspunkt des Katheters kann zum Beispiel mindestens ein unter Berücksichtigung der Struktur und/oder Anatomie und/oder Funktion des Gebiets sowie der Katheterplatzierungsrichtlinien am besten geeignete oder bestmögliche Eintrittspunkt ausgewählt und dargestellt werden. Insbesondere können mehrere Eintrittspunkte dargestellt werden und zum Beispiel nur der am besten geeignete Eintrittspunkt oder die am besten geeigneten Eintrittspunkte besonders hervorgehoben oder angezeigt werden.

Aus dem ermittelten mindestens einen möglichen Eintrittspunkt können nun beispielsweise korrespondierende oder zugehörige Zielpunkte ermittelt werden, wobei vorzugsweise die Zielpunkte bestimmt und angezeigt werden, welche innerhalb des zuvor angegebenen Zielgebiets oder den zuvor angegebenen Zielgebieten liegen oder um einen bestimmten oder vorgegebenen Bereich um die Zielgebiete liegen. Auch können aus den ermittelten Zielpunkten nur bestimmte, zum Beispiel die innerhalb des vorgegebenen Zielgebiets liegenden oder am besten geeigneten Zielpunkte, oder auch nur der am besten geeignete Zielpunkt angezeigt oder hervorgehoben oder dargestellt werden.

Auch können Trajektorien oder Bewegungs- oder Platzierungsvorschriften oder Bewegungsabläufe ermittelt und dargestellt werden, die angeben oder beschreiben, wie der Katheter z.B. von einem bekannten oder angegebenen mindestens einen Eintrittsgebiet oder Eintrittspunkt zu dem mindestens einen ermittelten Zielgebiet oder Zielpunkt bewegt werden kann. Dabei können die Bewegungs- oder Platzierungsvorschriften oder Bewegungsabläufe insbesondere angeben, wie der Katheter bestmöglich zwischen dem Eintrittsgebiet und dem Zielgebiet, vorzugsweise unter Berücksichtigung der Struktur und/oder der Anatomie des Gebiets und der Katheterplatzierungsrichtlinien, bewegt oder platziert werden kann.

Umgekehrt können die Trajektorien oder Bewegungs- oder Platzierungsvorschriften oder Bewegungsabstände auch beschreiben, wie der Katheter bestmöglich zwischen dem mindestens einen angegebenen oder vorgegebenen Zielgebiet und dem mindestens einen ermittelten Eintrittsgebiet bewegt oder platziert werden kann.

Die Bewegungs- oder Platzierungsvorschriften können vorzugsweise zusammen mit dem vorgegebenen oder ermittelten Eintrittsgebiet oder Zielgebiet und/oder mit den funktionellen Daten und/oder Struktur- und/oder Anatomiedaten des Gebiets und/oder mit den Katheterplatzierungsrichtlinien angezeigt oder insbesondere grafisch dargestellt werden. Zum Beispiel können die Bewegungs- oder Platzierungsvorschriften als Koordinaten ausgegeben werden oder als grafische Darstellung, wie als Pfeile oder Bewegungsvektoren, beispielsweise in oder mit den Struktur- und/oder Anatomiedaten des Gebiets angezeigt oder dargestellt werden.

Bevorzugt ist nach der Durchführung des erfindungsgemäßen Verfahrens der mindestens eine Katheter so platziert, dass minimal ein Abstand oder ein minimierter Abstand zu der Oberfläche des Gebiets, wie zu der Gehirnoberfläche oder zu charakteristischen Bereichen des Gebiets, wie Kanälen, Aushöhlungen, Sulci oder Ventrikeln, vorliegt, der den vorgegebenen Platzierungsrichtlinien genügt

Vorzugsweise werden die Katheterplatzierungsrichtlinien aus der europäischen Patentschrift EP 1 591 074 verwendet. Bevorzugt werden drei Katheterplatzierungsrichtlinien verwendet. Zum einen sollte der Abstand des Katheters oder der Katheterspitze oder des Katheterabgabeabschnittes oder sollten die Abstände der mehreren oder weiteren Katheter oder Katheterspitzen oder Katheterabgabeabschnitte von der Oberfläche des Gebiets, in welches der oder die Katheter eingesetzt oder eingeführt wurden, zwischen 1 und 4 cm, insbesondere 2,5 cm betragen. Weiter sollten die Abstände mehrerer eingesetzter Katheter oder Katheterspitzen oder Katheterabgabeabschnitte zueinander mindestens 1 cm, bevorzugt 3 cm, von Kathetern oder Katheterspitzen oder Katheterabgabeabschnitten anderer Katheter entfernt sein. Auch sollte der Abstand des Katheters oder der Katheterspitze oder des Katheterabgabeabschnittes oder bei dem Einsatz mehrerer Katheter bevorzugt der Abstand aller Katheter oder aller Katheterspitzen oder aller Katheterabgabeabschnitte von bestimmten, charakteristischen Abschnitten des Gebiets, wie bestimmten Gewebeabschnitten, insbesondere von Rinnen oder Furchen oder Sulci des Gebiets oder Gewebes und/oder von Kammern oder Ventrikeln des Gebiets oder Gewebes und/oder von Aushöhlungen oder Höhlen oder Löchern oder Kavitäten des Gebiets oder Gewebes, zwischen 2 und 15 mm, insbesondere 10 mm, betragen. Auch können minimal mögliche Abstände des Katheters zu charakteristischen Bereichen oder minimal mögliche Abstände der Katheter zueinander als Katheterplatzierungsrichtlinien verwendet werden.

Erfindungsgemäß können die Platzierungsrichtlinien auch dynamisch berechnet werden und sie können veränderlich in Abhängigkeit beispielsweise von der jeweiligen anatomischen und/oder funktionellen und/oder strukturellen Position des Katheters und/oder des Zielgebietes und/oder des Eintrittsgebietes veränderlich sein. Weiterhin können die Platzierungsrichtlinien den jeweiligen Kathetertypen den Infusionsparametern und/oder den Infusatparameter angepasst werden.

Insbesondere wird bei dem erfindungsgemäßen Verfahren als Gebiet, in welchem der Katheter oder die Katheter platziert werden soll bzw. sollen, ein Gewebe oder Teile eines Gewebes, wie ein Gehirn oder eine Gehirnregion, verwendet. Dabei können mittels eines medizintechnischen bildgebenden Verfahrens, wie Computertomographie, Kernspintomographie, Ultraschall, Positronen-Emissions-Tomographie oder SPECT-Tomographie, vor oder während des erfindungsgemäßen Verfahrens funktionelle Daten und/oder Struktur- und/oder Anatomiedaten des Gebiets oder des Gewebes aufgenommen werden. Somit können zum Beispiel Informationen über bestimmte Gewebeteile oder Gewebeabschnitte und/oder über Gebiete von durch Operationen entfernte Gewebeteile und/oder Gebiete von Gewebemissbildungen, Gewebeabnormitäten oder Gewebeanomalien, wie Ödemen, und/oder über charakteristische bestimmte anatomische Gebiete, wie den Gehirnstamm, vor der Durchführung des erfindungsgemäßen Verfahrens ermittelt werden und damit während der Durchführung des Verfahrens bekannt sein oder sie können während oder als Teil des erfindungsgemäßen Verfahrens ermittelt werden. Auch können als funktionelle Daten und/oder Struktur- und/oder Anatomiedaten Informationen über die Oberfläche des Gebiets oder Gewebes und/oder über Rinnen oder Furchen, Gyri und Sulci des Gebiets oder Gewebes, insbesondere Furchen zwischen den Hirnwindungen, und/oder über Kammern oder Ventrikel des Gebiets oder Gewebes und/oder über Aushöhlungen oder Höhlen oder Löcher oder Kavitäten des Gebiets oder Gewebes verwendet werden, die z.B. bekannt sein können oder vor oder während der Durchführung des erfindungsgemäßen Verfahrens ermittelt werden können. Mit Hilfe der gewonnenen oder bekannten funktionellen Daten und/oder Struktur- und/oder Anatomiedaten kann zum Beispiel die Ausbreitung oder Verteilung eines Infusionsmittels in dem Gebiet oder Gewebe bestimmt oder präzisiert oder vorhergesagt werden oder mittels der Struktur- und/oder Anatomiedaten kann die Einhaltung der Katheterplatzierungsrichtlinien überwacht oder gesteuert werden.

Die Erfindung bezieht sich des Weiteren auf ein Computerprogramm, welches, wenn es in einen Computer geladen wird oder auf einem Computer läuft, ein wie oben beschriebenes Verfahren durchführt. Weiterhin bezieht sich die Erfindung auf ein Programmspeichermedium oder ein Computerprogrammprodukt mit einem solchen Programm.

Die erfindungsgemäße Vorrichtung zur Bestimmung und Darstellung möglicher Eintritts- und/oder Zielgebiete für Katheterplatzierungen in einem Gebiet auf der Basis von funktionellen Daten und/oder Struktur- und/oder Anatomiedaten des Gebiets umfasst eine Dateneingabevorrichtung, wie eine Tastatur, eine Maus oder einen Touchscreen, mittels welcher z.B. mindestens ein Eintrittsgebiet eines Katheters oder mindestens ein Zielgebiet eines Katheters zum Beispiel über Koordinaten oder durch Auswahl eines Gebiets oder Kennzeichnung eines Gebiets auf einem Bildschirm oder Touchscreen eingegeben oder ausgewählt werden kann.

Das ausgewählte Eintrittsgebiet oder Zielgebiet oder die gewählten Gebiete können zum Beispiel farblich gekennzeichnet oder andersartig grafisch hervorgehoben oder gekennzeichnet oder dargestellt werden. Auch können zum Beispiel benutzerdefinierte Katheterplatzierungsrichtlinien über die Dateneingabevorrichtung in die erfindungsgemäße Vorrichtung eingegeben werden oder es können vorgegebene oder festgelegte Katheterplatzierungsrichtlinien über die Dateneingabevorrichtung individuell geändert werden. Auch können zum Beispiel mittels eines medizintechnischen bildgebenden Verfahrens, wie mittels eines Computer-Tomographen, Kernspin-Tomographen, Ultraschall-Tomographen, Positron-Emissions-Tomographen oder eines SPECT-Tomographen aufgenommene funktionelle Daten und/oder Struktur- und/oder Anatomiedaten des Gebiets über die Dateneingabevorrichtung in die erfindungsgemäße Vorrichtung eingegeben werden und zum Beispiel in einer Datenbank abgelegt oder gespeichert werden.

In einer Recheneinheit, wie einem Prozessor oder einem Computer, welche mit der Dateneingabevorrichtung verbunden sein kann, kann, basierend auf dem angegebenen oder ausgewählten Zielgebiet oder den Zielgebieten des Katheters, unter Berücksichtigung der in der Datenbank gespeicherten oder von einem Benutzer eingegebenen Katheterplatzierungsrichtlinien und der zum Beispiel in der Datenbank gespeicherten oder von einem Benutzer eingegebenen funktionellen Daten und/oder Struktur- und/oder Anatomiedaten des Gebiets oder Gewebes mindestens ein mögliches Eintrittsgebiet des Katheters berechnet werden.

Auch kann zum Beispiel von der Recheneinheit ermittelt werden, dass kein mögliches Eintrittsgebiet zu dem angegebenen Zielgebiet existiert. Auch kann einem Benutzer angezeigt werden, um wie viel berechnete Eintrittsgebiete von Eintrittsgebieten abweichen, die als mögliche oder geeignete Eintrittsgebiete unter Berücksichtigung der Katheterplatzierungsrichtlinien und der Struktur- und/oder Anatomiedaten verwendet werden können. Zum Beispiel kann ein Signalton oder eine grafische Ausgabe erfolgen, welche anzeigt, dass kein möglicher Eintrittspunkt ermittelt werden konnte.

Auch können mittels der Recheneinheit zu einem angegebenen oder vorgegebenen Eintrittsgebiet, welches zum Beispiel von einem Benutzer über eine Dateneingabevorrichtung ausgewählt wurde, mit Hilfe der bekannten oder ermittelten Struktur und/oder Funktion und/oder Anatomie des Gebiets oder Gewebes und mit Hilfe der gespeicherten oder eingegebenen Katheterplatzierungsrichtlinien ein zugehöriges oder mehrere zugehörige Zielgebiete ermittelt werden.

Die erfindungsgemäße Vorrichtung weist ferner eine Datenbank auf, welche mit der Recheneinheit verbunden oder in die Recheneinheit integriert sein kann, in welcher oder in welche Katheterplatzierungsrichtlinien gespeichert sein bzw. gespeichert werden können und/oder in welcher oder in welche zum Beispiel individuelle funktionelle Daten und/oder Struktur- und/oder Anatomiedaten verschiedener Gebiete oder Gewebe oder Gehirne abgelegt sein bzw. abgelegt werden können. Diese Daten können von der Recheneinheit ausgelesen und zur Ermittlung der Zielgebiete oder Zielpunkte oder Eintrittsgebiete oder Eintrittspunkte verwendet werden.

Auch umfasst die erfindungsgemäße Vorrichtung eine Datenausgabevorrichtung, wie einen Bildschirm oder einen Touchscreen, welche auch anstelle oder zusätzlich zu einer Dateneingabevorrichtung, wie einer Tastatur, als Dateneingabevorrichtung verwendet werden kann. Vorzugsweise ist die Datenausgabevorrichtung mit der Recheneinheit und/oder mit der Datenbank verbunden, so dass von der Datenausgabevorrichtung die funktionellen Daten und/oder Struktur- und/oder Anatomiedaten zum Beispiel grafisch dargestellt werden können und/oder die vorgegebenen oder ausgewählten Katheterplatzierungsrichtlinien, insbesondere innerhalb der grafisch dargestellten funktionellen Daten und/oder Struktur- und/oder Anatomiedaten, angezeigt werden können und/oder das mindestens eine angegebene oder ermittelte Zielgebiet oder das mindestens eine angegebene oder ermittelte Eintrittsgebiet, insbesondere in der Darstellung der Struktur, Funktion und Anatomie des Gebiets oder Gewebes, angezeigt werden können. Zum Beispiel können die Katheterplatzierungsrichtlinien oder die angegebenen oder ermittelten Eintrittsgebiete oder Zielgebiete farbig in der Struktur und/oder der Anatomie des Gebiets hervorgehoben werden, so dass ein Benutzer die Gebiete leicht auswählen und/oder erkennen kann.

Auch kann die erfindungsgemäße Vorrichtung ein medizintechnisches Trackingsystem aufweisen, welches die Position des Katheters oder der Katheter und die Position des Gebiets oder des Gewebes ermitteln kann. Das Trackingsystem ist vorzugsweise mit der Recheneinheit verbunden oder in die Recheneinheit integriert, so dass das Trackingsystem der Recheneinheit die Informationen über die Position des Katheters oder der Katheter und über die Position des Gebiets oder des Gewebes übermitteln kann. Die Recheneinheit kann die erhaltenen Informationen über die Position der Katheter und des Gebiets mit den Informationen über die Struktur und/oder die Anatomie des Gebiets korrelieren oder in Verbindung setzen oder mit den Informationen über die Eintrittsgebiete oder Zielgebiete in Verbindung setzen und die korrelierten Informationen auf der Datenausgabevorrichtung darstellen.

Die Erfindung wird nachfolgend anhand bevorzugter Ausführungsbeispiele beschrieben werden. Es zeigen:

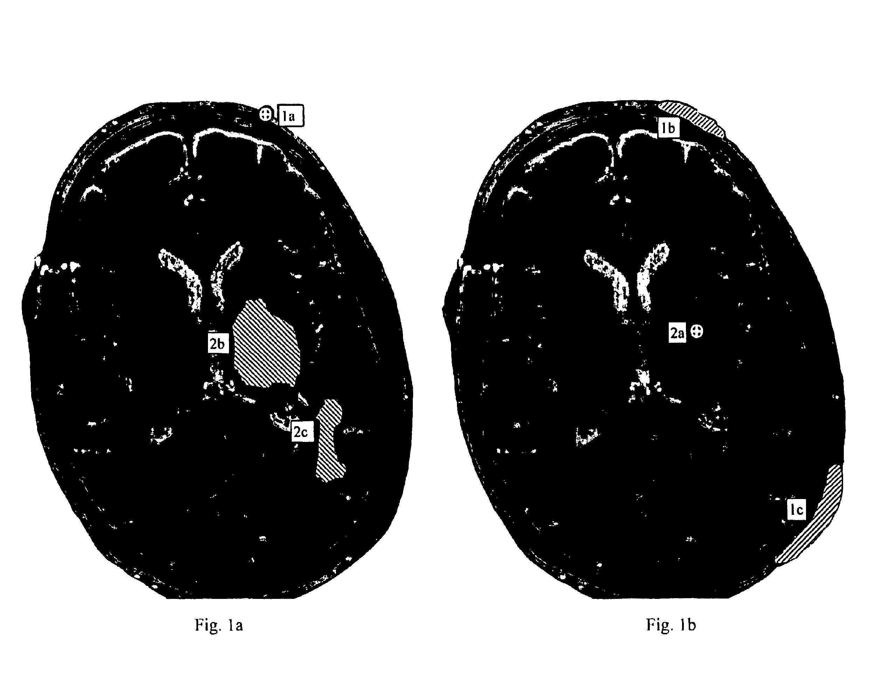

- Figur 1 a: eine erste Ausführungsform der vorliegenden Erfindung, bei welcher aus einem vorgegebenen Eintrittspunkt eines Katheters unter Berücksichtigung von Katheterplatzierungsrichtlinien mögliche Zielgebiete ermittelt werden; und
- Figur 1 b: eine weitere Ausführungsform der vorliegenden Erfindung, bei welcher aus einem vorgegebenen Zielpunkt des Katheters mögliche Eintrittsgebiete unter Berücksichtigung von Katheterplatzierungsrichtlinien ermittelt werden.

Figur 1 a zeigt eine erste Ausführungsform der vorliegenden Erfindung, bei welcher die Struktur und Anatomie eines Gehirns aufgenommen wurde und dargestellt wird und in der dargestellten Struktur und Anatomie des Gehirns ein Eintrittspunkt 1 a, z.B. von einem Benutzer vorgegeben wird und in der Struktur- und Anatomiedarstellung des Gehirns angezeigt und dargestellt wird. Aus dem angegebenen Eintrittspunkt 1a können mögliche Zielgebiete 2b, 2c des Katheters ermittelt werden, die z.B. so liegen, dass beim eingeführten oder eingesetzten Katheter Katheterplatzierungsrichtlinien und die Struktur und/oder die Anatomie des Gehirns berücksichtigt werden. Die Katheterplatzierungsrichtlinien können dabei insbesondere der Abstand der Katheterspitze oder des Katheterabgabeabschnitts von der Oberfläche des Gehirns oder Abstände des Katheters, der Katheterspitze oder des Katheterabgabeabschnittes von charakteristischen Regionen des Gehirns, wie Kavitäten oder Aushöhlungen, Sulzi oder Ventrikeln sein. Die unter Berücksichtigung der Katheterplatzierungsrichtlinien und der individuellen Struktur und Anatomie des Gehirns ermittelten Zielgebiete 2b, 2c können in den aufgenommenen und dargestellten Struktur- und Anatomiedaten des Gehirns dargestellt werden, so dass ein Benutzer, wie ein Arzt oder Chirurg, den am besten geeigneten oder bestmöglichen Zielbereich 2b, 2c auswählen kann. Auch können Bewegungs- oder Platzierungsvorschriften ermittelt und z.B. in den dargestellten Struktur- und Anatomiedaten des Gehirns angezeigt werden, die z.B. durch Vektoren angeben, wie die genaue Bewegung des Katheters gestaltet sein soll, um den Katheter unter Berücksichtigung der Anatomie und Struktur sowie der Katheterplatzierungsrichtlinien in dem Gehirn platzieren zu können.

Figur 1b zeigt eine weitere Ausführungsform der vorliegenden Erfindung, bei welcher umgekehrt zur ersten Ausführungsform, ein Zielpunkt 2a, z.B. von einem Benutzer, vorgegebenen wird und unter Berücksichtigung der Katheterplatzierungsrichtlinien und unter Berücksichtigung der individuellen Struktur und Anatomie des Gehirns am besten geeignete oder zugehörige Eintrittsgebiete 1b, 1c ermittelt werden, durch welche der Katheter in das Gehirn eingeführt und eingesetzt werden kann.

## Patentansprüche

1. Verfahren zur Bestimmung und Darstellung möglicher Eintritts- und/oder Zielgebiete (1b, 1 c, 2b, 2c) für Katheterplatzierungen in einem Gebiet auf der Basis von funktionellen Daten und/oder Struktur- und/oder Anatomiedaten des Gebiets, mit folgenden Schritten:
- es wird mindestens ein Eintrittsgebiet (1a) des Katheters oder mindestens ein Zielgebiet (2a) des Katheters angegeben;
- es wird basierend auf dem mindestens einen angegebenen Eintrittsgebiet (1a) des Katheters unter Berücksichtigung von vorgegebenen Katheterplatzierungsrichtlinien und der funktionellen Daten und/oder Struktur- und/oder Anatomiedaten des Gebiets mindestens ein Zielgebiet (2b, 2c) des Katheters ermittelt oder es wird basierend auf dem mindestens einen angegebenen Zielgebiet (2a) des Katheters unter Berücksichtigung von vorgegebenen Katheterplatzierungsrichtlinien und der funktionellen Daten und/oder Struktur- und/oder Anatomiedaten des Gebiets mindestens ein Eintrittsgebiet (1b, 1c) des Katheters ermittelt; und
- es werden die funktionellen Daten und/oder Struktur- und/oder Anatomiedaten mit dem mindestens einen angegebenen Eintrittsgebiet (1a) und dem mindestens einen ermittelten Zielgebiet (2b, 2c) dargestellt oder es werden die funktionellen Daten und/oder Struktur- und/oder Anatomiedaten mit dem mindestens einen angegebenen Zielgebiet (2a) und dem mindestens einen ermittelten Eintrittsgebiet (1b, 1c) dargestellt;
**dadurch gekennzeichnet, dass**
die Katheterplatzierungsrichtlinien Kriterien hinsichtlich vorgegebener oder festgelegter Abstandscharakteristika des Katheters zum charakteristischen Gebieten oder Kriterien hinsichtlich vorgegebener oder festgelegter Abstandscharakteristika mehrerer Katheter zueinander oder zu charakteristischen Gebieten sind.

2. Verfahren nach dem vorhergehenden Anspruch, wobei zusätzlich zu dem mindestens einen ermittelten Zielgebiet (2b, 2c) des Katheters oder dem mindestens einen ermittelten Eintrittsgebiet (1b, 1c) des Katheters die ermittelten funktionellen Daten und/oder Struktur- und/oder Anatomiedaten mit den vorgegebenen Katheterplatzierungsrichtlinien dargestellt werden.

3. Verfahren nach einem der vorhergehenden Ansprüche, wobei als Eintrittsgebiet (1a, 1b, 1 c) ein Eintrittspunkt angegeben und/oder ermittelt wird.

4. Verfahren nach einem der vorhergehenden Ansprüche, wobei als Zielgebiet (2a, 2b, 2c) ein Zielpunkt angegeben und/oder ermittelt wird.

5. Verfahren nach einem der vorhergehenden Ansprüche, wobei zunächst mindestens ein Eintrittsgebiet (1a) des Katheters angegeben wird, unter Berücksichtigung der vorgegebenen Katheterplatzierungsrichtlinien und der funktionellen Daten und/oder Struktur- und/oder Anatomiedaten des Gebiets mindestens ein möglicher Zielpunkt des Katheters ermittelt wird, wobei insbesondere der am besten geeignete Zielpunkt angezeigt wird.

6. Verfahren nach dem vorhergehenden Anspruch, wobei nach Ermittlung des mindestens einen möglichen Zielpunkts, mindestens ein zu dem ermittelten möglichen Zielpunkt korrespondierender Eintrittspunkt unter Berücksichtigung der Katheterplatzierungsrichtlinien innerhalb des angegebenen Eintrittsgebiets (1a) ermittelt wird, wobei insbesondere der am besten geeignete Eintrittspunkt angezeigt wird.

7. Verfahren nach einem der vorhergehenden Ansprüche, wobei zunächst mindestens ein Zielgebiet (2a) des Katheters angegeben wird, unter Berücksichtigung der vorgegebenen Katheterplatzierungsrichtlinien und der funktionellen Daten und/oder Struktur- und/oder Anatomiedaten des Gebiets mindestens ein möglicher Eintrittspunkt des Katheters ermittelt wird, wobei insbesondere der am besten geeignete Eintrittspunkt angezeigt wird.

8. Verfahren nach dem vorhergehenden Anspruch, wobei nach Ermittlung des mindestens einen möglichen Eintrittspunkts, mindestens ein zu dem ermittelten möglichen Eintrittspunkt korrespondierender Zielpunkt innerhalb des angegebenen Zielgebiets (2a) unter Berücksichtung der Katheterplatzierungsrichtlinien ermittelt wird, wobei insbesondere der am besten geeignete Zielpunkt angezeigt wird.

9. Verfahren nach einem der vorhergehenden Ansprüche, wobei die zur Bestimmung des geeigneten Zielgebiets oder der Zielgebiete bei vorgegebenem Eintrittspunkt oder vorgegebenen Eintrittsgebiet verwendeten Katheterplatzierungsrichtlinien abhängig sind von der anatomischen und/oder funktionellen und/oder strukturellen Eigenschaft des Eintrittspunktes oder des Eintrittsgebiets und/oder des jeweiligen Zielpunktes oder des Zielgebiets und/oder des Gebietes entlang der Trajektorie des Katheters.

10. Verfahren nach einem der vorhergehenden Ansprüche, wobei die zur bestimmung des geeigneten Eintrittsgebiets oder der Zielgebiete bei vorgegebenem Zielpunkt oder vorgegebenem Zielgebiet verwendeten Katheterplatzierungsrichtlinien abhängig sind von der anatomischen und oder funktionellen und oder strukturellen Eigenschaft des Zielpunktes oder des Zielgebiets und/oder des jeweiligen Eintrittspunktes oder des Eintrittsgebiets und/oder des Gebietes entlang der Trajektorie des Katheters.

11. Verfahren nach einem der vorhergehenden Ansprüche, wobei die Katheterplatzierungsrichtlinien abhängig sind von den jeweiligen Katheterparametern und/oder Infusionsparametern und/oder weiteren Treiberparametern und/oder Infusatparametern, wobei die Verwendung mehrerer aufeinanderfolgender Infusionen mit gleichen und/oder unterschiedlichen Infusaten betrachtet werden kann.

12. Verfahren nach einem der vorhergehenden Ansprüche wobei die Katheterplatzierungsrichtlinien abhängig sind von bereits definierten Kathetertrajektorien und/oder von der zu erwartenden Gebietsabdeckung der durch bereits definierte Kathetertrajektorien zu infundierenden Flüssigkeiten.

13. Verfahren nach einem der vorhergehenden Ansprüche, wobei basierend auf den vorgegebenen Katheterplatzierungsrichtlinien und den funktionellen Daten und/oder Struktur- und/oder Anatomiedaten des Gebiets Platzierungsvorschriften ermittelt werden, die angeben, wie der Katheter zwischen dem mindestens einen angegebenen oder ermittelten Eintrittsgebiet (1a, 1b, 1c) und dem mindestens einen angegebenen oder ermittelten Zielgebiet (2a, 2b, 2c) unter Einhaltung der Katheterplatzierungsrichtlinien platziert werden kann.

14. Verfahren nach einem der vorhergehenden Ansprüche, wobei als Katheterplatzierungsrichtlinien verwendet werden:
- die Abstände der Katheter oder der Katheterspitzen oder der Katheterabgabeabschnitte von der Oberfläche des Gebiets, wobei die Abstände insbesondere zwischen 1 und 4 cm betragen; und/oder
- die Abstände mehrerer Katheter oder Katheterspitzen oder Katheterabgabeabschnitte voneinander, wobei die Abstände mindestens 1 cm, insbesondere 3 cm, betragen; und/oder
- die Abstände der Katheter oder der Katheterspitzen oder der Katheterabgabeabschnitte von bestimmten Gewebeabschnitten, insbesondere von Rinnen oder Furchen oder Sulci des Gebiets und/oder von Kammern oder Ventrikeln des Gebiets und/oder von Aushöhlungen oder Höhlen oder Löchern oder Kavitäten des Gebiets, wobei die Abstände insbesondere zwischen 2 und 15 mm betragen.

15. Verfahren nach einem der vorhergehenden Ansprüche, wobei als Gebiet ein Gewebe, insbesondere ein Gehirn oder eine Gehirnregion, verwendet wird.

16. Verfahren nach einem der vorhergehenden Ansprüche, wobei als funktionelle Daten und/oder Struktur- und/oder Anatomiedaten Informationen über Gewebeteile und/oder über Gebiete von durch Operationen entfernte Gewebeteile und/oder über Gebiete von Gewebemissbildungen, Gewebeabnormitäten oder Gewebeanomalien, insbesondere Ödeme, und/oder über anatomische Gebiete, insbesondere den Gehirnstamm, verwendet und/oder ermittelt werden.

17. Verfahren nach einem der vorhergehenden Ansprüche, wobei als funktionelle Daten und/oder Struktur- und/oder Anatomiedaten Informationen über die Oberfläche des Gebiets und/oder über Rinnen oder Furchen, Sulci oder Gyri des Gebiets, insbesondere Furchen zwischen den Hirnwindungen, und/oder über Kammern oder Ventrikel des Gebiets und/oder über Aushöhlungen oder Höhlen oder Löcher oder Kavitäten des Gebiets verwendet und/oder ermittelt werden.

18. Computerprogramm, welches, wenn es auf einem Computer läuft oder in einen Computer geladen ist, das Verfahren nach einem der vorhergehenden Ansprüche durchführt.

19. Programmspeichermedium oder Computerprogrammprodukt mit dem Computerprogramm nach dem vorhergehenden Anspruch.

20. Vorrichtung zur Bestimmung und Darstellung möglicher Eintritts- und/oder Zielgebiete für Katheterplatzierungen in einem Gebiet auf der Basis von funktionellen Daten und/oder Struktur- und/oder Anatomiedaten des Gebiets, mit:
- einer Dateneingabevorrichtung zur Eingabe mindestens eines Eintrittsgebiets (1a) eines Katheters oder mindestens eines Zielgebiets (2a) des Katheters;
- einer Recheneinheit, welche mit der Dateneingabevorrichtung verbunden ist, zur Ermittlung mindestens eines Zielgebiets (2b, 2c) des Katheters unter Berücksichtigung von vorgegebenen Katheterplatzierungsrichtlinien und der funktionellen Daten und/oder Struktur- und/oder Anatomiedaten und des mindestens einen angegebenen Eintrittsgebiets (1a), oder zur Ermittlung mindestens eines Eintrittsgebiets (1 b, 1 c) des Katheters unter Berücksichtigung von vorgegebenen Katheterplatzierungsrichtlinien und der funktionellen Daten und/oder Struktur- und/oder Anatomiedaten und des mindestens einen angegebenen Zielgebiets (2a);
- einer Datenbank, in welcher Katheterplatzierungsrichtlinien und/oder funktionelle Daten und/oder Struktur- und/oder Anatomiedaten ablegbar sind, wobei die Datenbank mit der Dateneingabevorrichtung und der Recheneinheit verbunden ist;
- einer Datenausgabevorrichtung, insbesondere einem Bildschirm, welche mit der Recheneinheit verbunden ist, zur Ausgabe der funktionellen Daten und/oder Struktur- und/oder Anatomiedaten und/oder der vorgegebenen Katheterplatzierungsrichtlinien und/oder des mindestens einen ermittelten Zielgebiets (2b, 2c) oder des mindestens einen ermittelten Eintrittsgebiets (1b, 1c);
**dadurch gekennzeichnet, dass**
die Katheterplatzierungsrichtlinien Kriterien hinsichtlich vorgegebener oder festgelegter Abstandscharakteristika des Katheters zu charakteristischen Gebieten oder Kriterien hinsichtlich vorgegebener oder festgelegter Abstandscharakteristika mehrerer Katheter zueinander oder zu charakteristischen Gebieten sind.

21. Vorrichtung nach dem vorhergehenden Anspruch, mit einem medizintechnischen Trackingsystem zur Bestimmung der Position des Katheters und der Position des Gebiets, wobei die Recheneinheit eingerichtet ist die Informationen über die Position des Katheters und über die Position des Gebiets mit den funktionellen Daten und/oder Struktur- und/oder Anatomiedaten zu korrelieren und die korrelierten Informationen auf der Datenausgabevorrichtung darstellbar sind.

22. Vorrichtung nach einem der zwei vorhergehenden Ansprüche, welche zur Ausführung des Verfahrens nach einem der Ansprüche 1 bis 17 eingerichtet ist.

## Claims

1. A method for determining and representing possible entry and/or target areas (1b, 1c, 2b, 2c) for positioning catheters in an area on the basis of functional data and/or structural and/or anatomical data of the area, comprising the following steps:
- at least one entry area (1 a) of the catheter or at least one target area (2a) of the catheter is indicated;
- at least one target area (2b, 2c) of the catheter is determined, based on the at least one indicated entry area (1a) of the catheter and taking into consideration specified catheter positioning guidelines and the functional data and/or structural and/or anatomical data of the area, or at least one entry area (1b, 1 c) of the catheter is determined, based on the at least one indicated target area (2a) of the catheter and taking into consideration specified catheter positioning guidelines and the functional data and/or structural and/or anatomical data of the area; and
- the functional data and/or structural and/or anatomical data are represented with the at least one indicated entry area (1a) and the at least one determined target area (2b, 2c), or the functional data and/or structural and/or anatomical data are represented with the at least one indicated target area (2a) and the at least one determined entry area (1b, 1 c),
**characterised in that**
- the catheter positioning guidelines are criteria relating to specified or defined distance characteristics of the catheter from characteristic areas or criteria relating to specified or defined distance characteristics of a number of catheters from each other or from characteristic areas.

2. The method according to the preceding claim, wherein in addition to the at least one determined target area (2b, 2c) of the catheter or the at least one determined entry area (1b, 1 c) of the catheter, the determined functional data and/or structural and/or anatomical data are represented with the specified catheter positioning guidelines.

3. The method according to any one of the preceding claims, wherein an entry point is indicated and/or determined as the entry area (1a, 1b, 1 c).

4. The method according to any one of the preceding claims, wherein a target point is indicated and/or determined as the target area (2a, 2b, 2c).

5. The method according to any one of the preceding claims, wherein at least one entry area (1 a) of the catheter is initially indicated, and at least one possible target point of the catheter is determined, taking into consideration the specified catheter positioning guidelines and the functional data and/or structural and/or anatomical data of the area, wherein in particular, the most suitable target point is shown.

6. The method according to the preceding claim, wherein once the at least one possible target point has been determined, at least one entry point corresponding to the determined possible target point is determined inside the indicated entry area (1a), taking into consideration the catheter positioning guidelines, wherein in particular the most suitable entry point is shown.

7. The method according to any one of the preceding claims, wherein at least one target area (2a) of the catheter is initially indicated, and at least one possible entry point of the catheter is determined, taking into consideration the specified catheter positioning guidelines and the functional data and/or structural and/or anatomical data of the area, wherein in particular, the most suitable entry point is shown.

8. The method according to the preceding claim, wherein once the at least one possible entry point has been determined, at least one target point corresponding to the determined possible entry point is determined inside the indicated target area (2a), taking into consideration the catheter positioning guidelines, wherein in particular the most suitable target point is shown.

9. The method according to any one of the preceding claims, wherein the catheter positioning guidelines used to determine the suitable target area or target area for a specified entry point or specified entry area are dependent on the anatomical and/or functional and/or structural characteristics of the entry point or entry area and/or of the respective target point or target area and/or of the area along the trajectory of the catheter.

10. The method according to any one of the preceding claims, wherein the catheter positioning guidelines used to determine the suitable entry area or entry areas for a specified target point or specified target area are dependent on the anatomical and/or functional and/or structural characteristics of the target point or target area and/or of the respective entry point or entry area and/or of the area along the trajectory of the catheter.

11. The method according to any one of the preceding claims, wherein the catheter positioning guidelines are dependent on the respective catheter parameters and/or infusion parameters and/or other driver parameters and/or infusate parameters, wherein the use of a number of consecutive infusions using the same and/or different infusates may be considered.

12. The method according to any one of the preceding claims, wherein the catheter positioning guidelines are dependent on catheter trajectories already defined and/or on the area coverage to be expected from the fluids to be infused by catheter trajectories already defined.

13. The method according to any one of the preceding claims, wherein positioning specifications are determined, based on the specified catheter positioning guidelines and the functional data and/or structural and/or anatomical data of the area, said positioning specifications indicating how the catheter can be positioned between the at least one indicated or determined entry area (1a, 1b, 1c) and the at least one indicated or determined target area (2a, 2b, 2c), in compliance with the catheter positioning guidelines.

14. The method according to any one of the preceding claims, wherein the catheter positioning guidelines used are:
- the distances between the catheter or the catheter tips or the catheter delivery sections and the surface of the area, wherein the distances are in particular between 1 and 4 cm; and/or
- the distances between a number of catheters or catheter tips or catheter delivery sections, wherein the distances are at least 1 cm, in particular 3 cm; and/or
- the distances between the catheters or the catheter tips or the catheter delivery sections and particular tissue sections, in particular grooves or furrows or sulci and/or chambers or ventricles and/or hollows or recesses or holes or cavities of the area, wherein the distances are in particular between 2 and 15 mm.

15. The method according to any one of the preceding claims, wherein a tissue, in particular a brain or brain region, is used as the area.

16. The method according to any one of the preceding claims, wherein information on tissue parts and/or on areas of tissue parts removed by operations and/or on areas of tissue deformities, tissue abnormalities or tissue anomalies, in particular oedemas, and/or on anatomical areas, in particular the brain stem, is used and/or determined as functional data and/or structural and/or anatomical data.

17. The method according to any one of the preceding claims, wherein information on the surface of the area and/or on grooves or furrows, sulci or gyri of the area, in particular furrows between the cerebral convolutions, and/or on chambers or ventricles of the area and/or on hollows or recesses or holes or cavities of the area is used and/or determined as functional data and/or structural and/or anatomical data.

18. A computer program which, when running on a computer or loaded onto a computer, performs the method according to any one of the preceding claims.

19. A program storage medium or computer program product comprising the computer program according to the preceding claim.

20. A device for determining and representing possible entry and/or target areas for positioning catheters in an area on the basis of functional data and/or structural and/or anatomical data of the area, comprising:
- a data input device for inputting at least one entry area (1a) of a catheter or at least one target area (2a) of a catheter;
- a computing unit, connected to the data input device, for determining at least one target area (2b, 2c) of the catheter, taking into consideration specified catheter positioning guidelines and the functional data and/or structural and/or anatomical data and the at least one indicated entry area (1 a), or for determining at least one entry area (1 b, 1 c) of the catheter, taking into consideration specified catheter positioning guidelines and the functional data and/or structural and/or anatomical data and the at least one indicated target area (2a);
- a database in which catheter positioning guidelines and/or functional data and/or structural and/or anatomical data can be archived, wherein the database is connected to the data input device and the computing unit;
- a data output device, in particular a screen, connected to the computing unit for outputting the functional data and/or structural and/or anatomical data and/or the specified catheter positioning guidelines and/or the at least one determined target area (2b, 2c) or the at least one determined entry area (1b, 1c);
**characterised in that**
- the catheter positioning guidelines are criteria relating to specified or defined distance characteristics of the catheter from characteristic areas or criteria relating to specified or defined distance characteristics of a number of catheters from each other or from characteristic areas.

21. The device according to the preceding claim, comprising a medical tracking system for determining the position of the catheter and the position of the area, wherein the computing unit is configured to correlate the information on the position of the catheter and on the position of the area with the functional data and/or structural and/or anatomical data, and wherein the correlated information can be represented on the data output device.

22. The device according to any one of the preceding two claims, which is configured to perform the method according to any one of claims 1 to 17.

## Revendications

1. Procédé pour déterminer et représenter des régions d'entrée et/ou régions cibles (1b, 1c, 2b, 2c) possibles pour des placements de cathéter dans une région, sur la base de données fonctionnelles et/ou structurelles et/ou anatomiques de la région, comportant les étapes suivantes:
- on indique au moins une région d'entrée (1a) du cathéter ou au moins une région cible (2a) du cathéter ;
- sur la base de la au moins une région d'entrée (1a) indiquée du cathéter, en tenant compte de directives prédéterminées de placement de cathéter et des données fonctionnelles et/ou structurelles et/ou anatomiques de la région, on détermine au moins une région cible (2b, 2c) du cathéter ou, sur la base de la au moins une région cible (2a) indiquée du cathéter, en tenant compte de directives prédéterminées de placement de cathéter et des données fonctionnelles et/ou structurelles et/ou anatomiques de la région, on détermine au moins une région d'entrée (1b, 1c) du cathéter ; et
- on représente les données fonctionnelles et/ou structurelles et/ou anatomiques avec la au moins une région d'entrée (1a) indiquée et la au moins une région cible (2b, 2c) déterminée, ou on représente les données fonctionnelles et/ou structurelles et/ou anatomiques avec la au moins une région cible (2a) indiquée et la au moins une région d'entrée (1b, 1c) déterminée ;
**caractérisé en ce que** les directives de placement de cathéter sont des critères relatifs à des caractéristiques prédéfinies ou fixées de distances du cathéter par rapport à des régions caractéristiques, ou des critères relatifs à des caractéristiques prédéfinies ou fixées de distances de plusieurs cathéters les uns par rapport aux autres ou par rapport à des régions caractéristiques.

2. Procédé selon la revendication précédente, dans lequel outre la au moins une région cible (2b, 2c) déterminée du cathéter ou la au moins une région d'entrée (1b, 1c) déterminée du cathéter, on représente les données fonctionnelles et/ou structurelles et/ou anatomiques déterminées avec les directives prédéterminées de placement de cathéter.

3. Procédé selon l'une des revendications précédentes, dans lequel on indique et/ou détermine un point d'entrée en tant que région d'entrée (1a, 1b, 1c).

4. Procédé selon l'une des revendications précédentes, dans lequel on indique et/ou détermine un point cible comme région cible (2a, 2b, 2c).

5. Procédé selon l'une des revendications précédentes, dans lequel on indique au moins d'abord une région d'entrée (1a) du cathéter, en tenant compte des directives prédéfinies de placement de cathéter et des données fonctionnelles et/ou structurelles et/ou anatomiques de la région, on détermine au moins un point cible possible du cathéter, le point cible qui convient le mieux étant en particulier indiqué.

6. Procédé selon la revendication précédente, dans lequel, après détermination d'au moins un point cible possible, on détermine au moins un point d'entrée correspondant au point cible possible déterminé, en tenant compte des directives de placement de cathéter à l'intérieur de la région d'entrée (1a) indiquée, le point d'entrée qui convient le mieux étant en particulier indiqué.

7. Procédé selon l'une des revendications précédentes, dans lequel on indique d'abord au moins une région cible (2a) du cathéter, en tenant compte des directives prédéfinies de placement de cathéter et des données fonctionnelles et/ou structurelles et/ou anatomiques de la région, on détermine au moins un point d'entrée possible du cathéter, le point d'entrée qui convient le mieux étant en particulier indiqué.

8. Procédé selon la revendication précédente, dans lequel, après détermination du au moins un point d'entrée possible, on détermine au moins un point cible correspondant au point d'entrée possible déterminé, à l'intérieur de la région cible (2a) indiquée, en tenant compte des directives de placement de cathéter, le point cible qui convient le mieux étant en particulier indiqué.

9. Procédé selon l'une des revendications précédentes, dans lequel les directives de placement de cathéter, utilisées pour déterminer la région cible appropriée ou les régions cibles, avec point d'entrée prédéfini ou région d'entrée prédéfinie, dépendent de la propriété anatomique et/ou fonctionnelle et/ou structurelle du point d'entrée ou de la région d'entrée et/ou du point cible respectif ou de la région cible et/ou de la région le long de la trajectoire du cathéter.

10. Procédé selon l'une des revendications précédentes, dans lequel les directives de placement de cathéter, utilisées pour déterminer la région d'entrée appropriée ou les régions cibles avec point cible prédéfini ou région cible prédéfinie, dépendent de la propriété anatomique et/ou fonctionnelle et/ou structurelle du point cible ou de la région cible et/ou du point d'entrée respectif ou de la région d'entrée et/ou de la région le long de la trajectoire du cathéter.

11. Procédé selon l'une des revendications précédentes, dans lequel les directives de placement de cathéter dépendent des paramètres respectifs du cathéter et/ou de paramètres de perfusion et/ou d'autres paramètres du produit propulseur et/ou de paramètres du produit de perfusion, l'utilisation de plusieurs perfusions successives avec des produits de perfusion identiques et/ou différentes pouvant être envisagée.

12. Procédé selon l'une des revendications précédentes, dans lequel les directives de placement de cathéter dépendent de trajectoires de cathéter déjà définies et/ou de la couverture de régions probables des liquides à perfuser à travers des trajectoires de cathéter déjà définies.

13. Procédé selon l'une des revendications précédentes, dans lequel, sur la base des directives de placement de cathéter prédéfinies et des données fonctionnelles et/ou structurelles et/ou anatomiques de la région, on détermine des prescriptions de placement qui indiquent comment le cathéter peut être placé entre la au moins une région d'entrée (1a, 1b, 1c) indiquée ou déterminée et la au moins une région cible (2a, 2b, 2c) indiquée ou déterminée, en respectant les directives de placement de cathéter.

14. Procédé selon l'une des revendications précédentes, dans lequel on utilise comme directives de placement de cathéter :
- les distances du cathéter ou des pointes de cathéter ou des segments de distribution de cathéter par rapport à la surface de la région, les distances étant en particulier comprises entre 1 et 4 cm ; et/ou
- les écartements de plusieurs cathéters ou pointes de cathéter ou segments de distribution de cathéter, les écartements étant d'au moins 1 cm, en particulier de 3 cm ; et/ou
- les distances des cathéters ou des pointes de cathéter ou des segments de distribution de cathéter par rapport à des segments de tissu déterminés, en particulier de conduits ou sillons de la région et/ou de chambres ou ventricules de la région et/ou de renfoncements ou cavernes ou trous ou cavités de la région, les distances étant comprises en particulier entre 2 et 15 mm.

15. Procédé selon l'une des revendications précédentes, dans lequel on utilise comme région un tissu, en particulier un cerveau ou une région du cerveau.

16. Procédé selon l'une des revendications précédentes, dans lequel on utilise et/ou détermine en tant que données fonctionnelles et/ou structurelles et/ou anatomiques, des informations relatives à des parties de tissu et/ou relatives à des régions de parties de tissu séparées par des opérations et/ou relatives à des régions de malformations de tissu, en particulier malformations de tissu ou anomalies de tissu, en particulier oedèmes, et/ou relatives à des régions anatomiques, en particulier de l'encéphale.

17. Procédé selon l'une des revendications précédentes, dans lequel on utilise et/ou détermine en tant que données fonctionnelles et/ou structurelles et/ou anatomiques, des informations relatives à la surface de la région et/ou relatives à des conduits ou sillons, ou circonvolutions de la région, en particulier aux sillons entre les circonvolutions du cerveau, et/ou relatives à des champs ou ventricules de la région et/ou relatives à des renfoncements ou cavernes ou trous ou cavités de la région.

18. Programme d'ordinateur qui, lorsqu'il tourne sur un ordinateur ou est chargé dans un ordinateur, exécute le procédé selon l'une des revendications précédentes.

19. Support de mémoire de programme ou produit de programme d'ordinateur avec le programme d'ordinateur selon la revendication précédente.

20. Dispositif pour déterminer et représenter des régions possibles d'entrée et/ou régions cibles pour des placements de cathéter dans une région, sur la base de données fonctionnelles et/ou structurelles et/ou anatomiques de la région, comportant
- un dispositif d'entrée de données pour introduire au moins une région d'entrée (1a) d'un cathéter ou au moins une région cible (2a) du cathéter ;
- une unité informatique, qui est reliée au dispositif d'entrée de données, pour déterminer au moins une région cible (2b, 2c) du cathéter en tenant compte de directives prédéfinies de placement de cathéter et des données fonctionnelles et/ou structurelles et/ou anatomiques et de la au moins une région d'entrée (1a) indiquée, ou pour déterminer au moins une région d'entrée (1b, 1c) du cathéter en tenant compte de directives prédéfinies de placement de cathéter et des données fonctionnelles et/ou données structurelles et/ou données anatomiques et de la au moins une région cible (2a) indiquée ;
- une banque de données dans laquelle peuvent être enregistrées des directives de placement de cathéter et/ou des données fonctionnelles et/ou structurelles et/ou anatomiques, la banque de données étant connectée au dispositif d'entrée de données et à l'unité informatique ;
- un dispositif de sortie de données, en particulier un écran, qui est relié à l'unité informatique, pour éditer les données fonctionnelles et/ou structurelles et/ou anatomiques et/ou les directives prédéfinies de placement de cathéter et/ou la au moins une région cible (2b, 2c) déterminée ou la au moins une région d'entrée (1b, 1c) déterminée ;
**caractérisé en ce que** les directives de placement de cathéter sont des critères relatifs à des caractéristiques prédéfinies ou fixées de distances du cathéter par rapport à des régions caractéristiques, ou des critères relatifs à des caractéristiques prédéfinies ou fixées de distances de plusieurs cathéters les uns par rapport aux autres ou par rapport à des régions caractéristiques.

21. Dispositif selon la revendication précédente, comportant un système de localisation de technique médicale pour déterminer la position du cathéter et la position de la région, l'unité informatique étant conçue pour corréler les informations relatives à la position du cathéter et relatives à la position de la région avec les données fonctionnelles et/ou structurelles et/ou anatomiques, et les informations corrélées pouvant être représentées sur le dispositif de sortie de données.

22. Dispositif selon l'une des deux revendications précédentes qui est conçu pour mettre en oeuvre le procédé selon l'une des revendications 1 à 17.
